# EUROPÄISCHE PATENTSCHRIFT

(11) **EP 0 307 857 B1**
(45) Veröffentlichungstag und Bekanntmachung des Hinweises auf die Patenterteilung: **09.08.1995**
(21) Anmeldenummer: 88114944.7
(22) Anmeldetag: 13.09.1988
(51) Int. Cl.: A61K 38/21, A61K 38/00, A61K 47/00

(54) **Stabilisierung von therapeutisch wirksamen Proteinen in pharmazeutischen Zubereitungen**
Stabilization of therapeutically active proteins in pharmaceutical preparations
Stabilisation de protéines thérapeutiquement actives dans des préparations pharmaceutiques

(30) Priorität: 17.09.1987 DE 3731255
(43) Veröffentlichungstag der Anmeldung: 22.03.1989
(73) Patentinhaber: BOEHRINGER INGELHEIM INTERNATIONAL GmbH, 55218 Ingelheim am Rhein (DE)
(72) Erfinder: Mundorf, Traute, Dr., A-1130 Wien (AT); Schnecker, Kurt, Dr., A-1170 Wien (AT)

(56) Entgegenhaltungen:
- EP-A- 0 177 342
- EP-A- 0 231 816
- EP-A- 0 233 629

## Beschreibung

Gegenstand der Erfindung sind pharmazeutische Zubereitungen für die topische Anwendung, enthaltend ein oder mehrere stabilisierte therapeutisch wirksame Proteine sowie gegebenenfalls übliche Hilfs-, Träger- und Zusatzstoffe. Die Erfindung betrifft auch ein Verfahren zur Herstellung derartiger pharmazeutischer Zubereitungen sowie die Verwendung physiologisch verträglicher hydrophober Substanzen zur Stabilisierung von Proteinen.

Eine der wesentlichen Forderungen bei der topischen Anwendung von therapeutisch wirksamen Proteinen richtet sich auf deren Stabilität in der pharmazeutischen Formulierung. Die Stabilität muß über einen ausreichend langen Zeitraum sowohl während der Lagerung bei Kühl- und bei Raumtemperatur als auch bei Körpertemperatur, außerdem über mehrere Stunden "in situ" gewährleistet sein. Hinsichtlich dieser Anforderungen konnten bisher keine voll befriedigenden Lösungen gefunden werden. Es wurden bereits verschiedene Substanzen zur Stabilisierung von Interferonen vorgeschlagen, wobei z.B. Hydroxyethylcellulose als Trägermaterial zur Herstellung von interferonhaltigen Gelen oder Salben eingesetzt wurde. Dabei traten jedoch unter Anwendungsbedingungen Aktivitätsverluste der Interferone auf, die nur durch Zusatz eines Proteaseinhibitors verringert werden konnten (EP-A-142345).

Für die Stabilisierung von Interferonen in Gelen, Salben, etc. wurden auch verschiedene Zuckeralkohole, ggf. gemeinsam mit Zuckersäuren oder deren Salzen, milden Reduktionsmitteln, anionischen Tensiden oder Kombinationen dieser Substanzen beschrieben (EP-A-80879).

Für die Stabilisierung von Proteinen und Polypeptiden wie Interferonen, insbesondere IFN-gamma, in parenteralen Applikationsformen wurde die Verwendung einer physikalisch und chemisch modifizierten Gelatine vorgeschlagen, vor allem unter dem Gesichtspunkt eines Ersatzes für Humanserumalbumin (EP-A-162332).

Die japanische veröffentlichte Patentanmeldung JP-A-61-277633 offenbart die Stabilisierung von Interferonen in Lösung mit bestimmten oberflächenaktiven Substanzen.

In der EP-A-135171 wird für Öl/Wasser-Mikroemulsionen Humanserumalbumin als geeigneter Stabilisator angeführt.

Für die topische Anwendung der synergistischen Kombination
IFN-beta/9-(1,3-Dihydroxy-2-propoxy-methyl)guanin (DHPG) gemäß der US-PS-4606917 in Form einer Salbe werden als Stabilisatoren Albumin, Dextrose und Puffersubstanzen vorgeschlagen.

Die therapeutische Zusammensetzung gemäß der EP-A-0233629 enthält neben einer antiviralen und einer Substanz mit Antitumor-Aktivität als physiologisch annehmbaren Träger u.a. "mineral oil" oder "petrolatum" in den für Trägerstoffen typisch hohen Anteilen. Von diesen Trägerstoffen wird gefordert, daß sie die Stabilität der wirksamen Substanzen nicht beeinträchtigen sollen.

Mit den bisher zur Stabilisierung von therapeutisch wirksamen Proteinen vorgeschlagenen Substanzen konnte, vor allem in Hydrogelen, eine stabilisierende Wirkung nicht im erforderlichen Maß erzielt werden.

Der Erfindung lag die Aufgabe zugrunde, topisch anzuwendende pharmazeutische Zubereitungen, insbesondere Hydrogele, bereitzustellen, die einen Stabilisator für therapeutisch wirksame Proteine enthalten, der neben seiner physiologischen Verträglichkeit sämtlichen Anforderungen genügt, die an derartige Formulierungen, vor allem im Hinblick auf die optimale Verfügbarkeit der Wirksubstanz und Erhaltung ihrer Aktivität sowie im Hinblick auf eine möglichst schonende Herstellungsweise, die der Empfindlichkeit der Proteine gegen Scherkräfte Rechnung trägt, gestellt werden.

Ausgehend von dieser Aufgabenstellung wurden verschiedene Substanzklassen auf ihre Tauglichkeit zur Lösung dieser Aufgabe untersucht. Dabei wurde überraschend festgestellt, daß hydrophobe Substanzen bereits in geringer Menge als Zusatzstoffe in feinster Verteilung, insbesondere Paraffinöle, eine stabilisierende Wirkung auf verschiedene therapeutisch wirksame Proteine zeigen, die der Wirkung der bisher vorgeschlagenen Substanzen überlegen ist. Dieses Ergebnis ist umso überraschender, als die zum Stand der Technik gehörenden topisch anzuwendenden pharmazeutischen Zubereitungen wie Salben, in denen hydrophobe Substanzen als Träger in entsprechend hohem Anteil eingesetzt werden, einen gesonderten Zusatz von Stabilisator erfordern.

Die Erfindung löst die gestellte Aufgabe dadurch, daß in pharmazeutischen Zubereitungen zur topischen Anwendung, insbesondere Hydrogelen, physiologisch verträgliche hydrophobe Substanzen, insbesondere Paraffinöle, zur Stabilisierung der therapeutisch wirksamen Proteine verwendet werden.

Gegenstand der Erfindung sind somit pharmazeutische Zubereitungen der eingangs erwähnten Art, enthaltend ein oder mehrere stabilisierte therapeutisch wirksame Proteine sowie übliche Hilfs-, Träger- und Zusatzstoffe, welche dadurch gekennzeichnet sind, daß sie als Stabilisator fein verteilt eine oder mehrere physiologisch verträgliche hydrophobe Substanzen, insbesondere Paraffinöl(e), in einer das Protein stabilisierenden Menge enthalten,die geringer ist als die Menge, in der die Substanz eine Trägerfunktion hat.

Mit Hilfe des in stabilisierender Menge vorgenommenen Zusatzes von hydrophoben Substanzen in feinverteilter Form werden pharmazeutische Zubereitungen erhalten, die unter Praxisbedingungen die Wirksubstanz über einen langen Zeitraum in aktiver Form verfügbar machen. Mit Hilfe der erfindungsgemäßen pharmazeutischen Zubereitungen bleibt bei Lagerung bei 4 - 8°C über einen Zeitraum von mindestens 12 Monaten die Aktivität des Proteins im wesentlichen unverändert erhalten. Ein weiterer Vorteil besteht darin, daß an derartige Formulierungen geringere Ansprüche hinsichtlich eines genau einzuhaltenden pH-Werts gestellt werden müssen, weil ein stabilisierender Zusatz hydrophober Substanzen die Empfindlichkeit der Proteine gegenüber pH-Wertschwankungen verringert.

Dieser Vorteil kommt vor allem dann zum Tragen, wenn Applikationen vorgesehen sind, die niedrigere pH-Werte bedingen, z.B. die Applikation im Vaginalbereich.

Die erfindungsgemäße pharmazeutische Zubereitung bietet darüberhinaus, wenn sie als Hydrogel vorliegt, den Vorteil, bei der Anwendung als äußerst angenehm empfunden zu werden. Durch die Anwesenheit der hydrophoben Substanz ist nämlich auch nach Eintrocknen des Gels die Geschmeidigkeit der aufgetragenen Schicht gegeben, was vor allem bei der Applikation im Lippenbereich besonders vorteilhaft ist.

Die vorteilhafte stabilisierende Wirkung hydrophober Substanzen auf Proteine ist möglicherweise auf hydrophobe Wechselwirkungen zurückzuführen, die bisher nicht oder in zu geringem Maße beachtet wurden. Bei der Stabilisierung von Proteinen gemäß dem Stand der Technik wurde offensichtlich von der Anwendung der beiden folgenden Funktionsprinzipien ausgegangen: a) Stabilisierung durch komplexe Bindung der Substanz an das Protein und damit sterische Fixierung des Proteinmoleküls; b) Bindung des freien Bulkwassers durch polare Substanzen und damit Stabilisierung des Proteins durch Beeinflussung seiner Hydrathülle.Die bei der vorliegenden Erfindung vermutlich zum Tragen kommenden hydrophoben Wechselwirkungen, wie sie etwa auch bei Micellarstrukturen auftreten, dürften hingegen eine Stabilisierung dadurch bewirken, daß die hydrophoben Teilbereiche des Proteins, die auf Grund der räumlichen Verteilung der hydrophoben und hydrophilen Aminosäurereste zustandekommen, an der Phasengrenzfläche Öl/Wasser fixiert werden, so daß die hydrophoben Bereiche in das Öltröpfchen, die hydrophilen Teile in die polare Phase ragen.

Als hydrophobe Substanzen kommen neben den bevorzugt verwendeten Paraffinölen höhere Fettsäuren wie Linolsäure und Palmitinsäure, oder höhere Alkohole wie Myristylalkohol, oder Fettsäureester wie Triglyceride oder modifizierte, z.B. polyoxyethylenierte und glykosylierte, Glyceride (Labrafil^{R}), einzeln oder in Mischung , in Betracht. Von den Paraffinölen sind Paraffinöl flüssig, dünnflüssig oder dickflüssig gemäß Ph. Eur. und USP oder Mischungen davon geeignet.Die hydrophoben Substanzen sind bevorzugt in einer Menge von 0,1 bis 3,0 % in der Zubereitung enthalten.

Um die Ausbildung einer feinen Verteilung des Stabilisators und die Beständigkeit dieser Verteilung zu gewährleisten, kann ein Zusatz von Emulgatoren vorgesehen sein. Die Menge richtet sich vor allem nach Art und Menge des Stabilisators, dem Träger sowie im Falle von Hydrogelen nach deren Viskosität; im allgemeinen beträgt sie maximal 1%. Als Emulgatoren kommen vor allem nicht-ionische wie Polysorbate (Polyoxyethylen(n)sorbitanmonolaurat, z.B. TweenR 20), Nonoxynol (Polyoxyethylen(n)nonylphenylether, z.B. TritonR N101, TritonR N111), Poloxamer (Polyethylenpoly-propylenglykol, PluronicR F68) in Betracht. Im Falle des Vorliegens der pharmazeutischen Zubereitung als Hydrogel bewirken die Emulgatoren neben der feinen Verteilung des Stabilisators auch eine bessere Spreitung der Gele.

Die erfindungsgemäßen pharmazeutischen Zubereitungen eignen sich für die Applikation von menschlichen und tierischen Proteinen wie den folgenden einschließlich deren strukturell ähnlichen bioaktiven Äquivalenten (unter derartigen Äquivalenten sind solche Proteine zu verstehen, die bei unterschiedlicher Aminosäuresequenz im wesentlichen gleiche biologische Wirkung aufweisen): Zytokine, z.B. Interferone wie huIFNalpha, huIFNbeta, huIFNgamma, huIFNomega, Hybridinterferone, tierische Interferone wie EqIFNbeta, EqIFNgamma, oder Lymphokine wie Interleukin-2, TNFbeta, oder Monokine wie Interleukin-1, TNFalpha; Wachstumsfaktoren, z.B. Epidermaler Wachstumsfaktor (EGF); Antikoagulantien, z.B. vascular-antikoagulierende Proteine (z.B. VAC alpha, VAC beta), Antithrombine; Fibrinolytika, z.B. tPA, Urokinase; antiallergisch wirkende Proteine, z.B. IgE-Bindungsfaktor; therapeutisch wirksame Enzyme, z.B.Lysozym, Superoxiddismutasen.

Die zum Einsatz kommenden Proteine können entweder natürlichen Ursprungs oder auf rekombinantem Weg hergestellt sein. Das Indikationsspektrum richtet sich nach der biologischen Aktivität des zu applizierenden Proteins; innerhalb des für das jeweilige Protein spezifischen Spektrums sind sämtliche Anwendungen möglich, die die topische Applikation der Wirksubstanz erfordern. Der Gehalt der pharmazeutischen Zubereitung an therapeutisch wirksamem Protein richtet sich selbstverständlich nach der Wirksamkeit des Proteins, den Erfordernissen der jeweiligen Indikation sowie der Applikationsform. Er kann einen breiten Mengenbereich umfassen.

Als Anwendungsformen sind insbesondere Hydrogele, Suppositorien und Anwendungsformen für den Vaginalbereich geeignet.

Die Verwendung von Hilfs-, Träger- und Zusatzstoffen richtet sich nach der jeweils gewählten Anwendungsform, wobei zu beachten ist, daß sie nach Art und Menge die Stabilität des Proteins nicht beeinträchtigen.

An Zusatzstoffen können die erfindungsgemäßen pharmazeutischen Zubereitungen Konservierungsmittel wie p-Hydroxybenzoesäureester (Nipa-Ester, Methylparaben), Sorbinsäure, Chlorhexidindigluconat, Benzalkoniumchlorid und Hexadecyltrimethylammoniumbromid, enthalten.

Um die Aufnahme des Wirkstoffes durch die Haut zu beschleunigen, können der pharmazeutischen Zubereitung Permeationsbeschleuniger wie Dimethylsulfoxid oder Tauroglykolsäure zugesetzt werden.

Als Hydrogelbildner sind u.a. Gelatine und Cellulosederivate wie Methylcellulose, Hydroxypropylcellulose und, besonders bevorzugt, Hydroxyethylcellulose, ferner synthetische Polymere wie Polyvinylalkohol geeignet. Art und Menge des verwendeten Hydrogelbildners bzw. der Mischungen davon richten sich nach der jeweils gewünschten Viskosität. Bezüglich der feinen Verteilung des Stabilisators ist in diesem Zusammenhang zu beachten, daß bei höherer Viskosität des Gels die Beständigkeit der Emulsion unter Umständen bereits durch den Gehalt an Hydrogelbildner in ausreichenden Maß gewährleistet ist und sich somit der Zusatz eines Emulgators erübrigen kann.
Die verwendeten Puffersysteme werden in Abhängigkeit vom pH-Optimum für das jeweilige Protein unter Abstimmung auf die jeweilige Applikation gewählt, in Frage kommen organische wie anorganische Puffer, z.B. Succinat-, Acetat- und Phosphatpuffer.

Der verwendete Träger richtet sich nach der Applikationsform, im Falle des Vorliegens der pharmazeutischen Zubereitung als Hydrogel ist der Träger Wasser.
Zu den gegebenenfalls vorgesehenen Zusatzstoffen zählen auch Feuchthaltesubstanzen wie Glycerin, Sorbit, 1,2-Propylenglykol, Butylenglykol sowie Polyole.

Bei den erfindungsgemäßen Zubereitungen in Form von Hydrogelen handelt es sich, bedingt durch den niedrigen Ölanteil, um sog. "niedrig gefüllte" Emulsionen, die bekanntlich leicht zum Brechen neigen. Im Hinblick auf die Beständigkeit einer solchen Emulsion kommt daher ihrer Herstellung besondere Bedeutung zu.

Um die möglichst schonende, mit relativ geringem technischem Aufwand verbundene Herstellung einer stabilen Emulsion, in der die Beständigkeit der feinen Verteilung des Stabilisators und damit seine Wirkung über einen langen Zeitraum gegeben ist, zu gewährleisten, wird bei der Herstellung der erfindungsgemäßen Zubereitungen, insbesondere von Hydrogelen, bevorzugt ein zweistufiges Verfahren angewendet.
Dabei wird in der ersten Stufe im System Wasser/Stabilisator/gegebenenfalls Emulgator eine Phasenumkehr von einer W/O-Emulsion zu einer O/W-Emulsion herbeigeführt und die dabei erhaltene feine Voremulsion mit der Hauptmenge der wässerigen Phase vereinigt.

Besonders bevorzugt wird wie folgt vorgegangen: Zunächst wird nach der sog. "kontinentalen" Methode eine Voremulsion hergestellt: der Emulgator wird im Paraffinöl verteilt und langsam Wasser bis zum Entstehen einer sehr groben W/O-Emulsion zugemischt. In diesem Stadium, das erfahrungsgemäß bei einem Wasseranteil von ca. 20-40 % erreicht wird, wird der Mischvorgang abgebrochen und die Emulsion kurz sedimentieren gelassen. Durch anschließendes erneutes Mischen und Wasserzugabe bis zu einem Anteil von ca. 50 % kippt die Emulsion unter Bildung einer feinen O/W-Emulsion. Im Zuge der zweiten Verfahrensstufe wird die erhaltene Voremulsion in die Pufferlösung eingerührt und dispergiert, wonach der Hydrogelbildner zugefügt und quellen gelassen wird. Der Zeitpunkt für die Zugabe der Proteinlösung ist nicht kritisch, bevorzugt erfolgt sie als letzter Verfahrensschritt. Mit dem erfindungsgemäß bevorzugten Verfahren werden äußerst stabile Emulsionen erhalten, die nach einem halben Jahr Lagerung bei Zimmertemperatur keine Tendenz zum Aufrahmen zeigten.

Bei kleineren Ansätzen sowie bei Verfügbarkeit von technisch aufwendigeren Homogenisatoren wie Düsenhomogenisatoren kann die Herstellung einer O/W-Emulsion auch in einem einzigen Verfahrensschritt ohne Herstellung einer Voremulsion erfolgen; das erfindungsgemäß bevorzugte Verfahren ermöglicht jedoch neben der Stabilität der Emulsion eine einfache, geringen Energie- und technischen Aufwand erfordernde und gleichzeitig schonende Herstellung.

Die folgenden Beispiele sollen die Erfindung an Hand von Hydrogelformulierungen mit IFN alpha, IFN gamma, TNF alpha, TNF beta, Lysozym oder VAC alpha als therapeutisch wirksames Protein illustrieren:

### Beispiel 1

100 Gramm Gel enthielten:

| | |
|---|---|
| IFN gamma | 0,1 g |
| Methylparaben | 0,2 g |
| Natriumdihydrogenphosphat-Monohydrat | 0,05 g |
| di-Kaliumhydrogenphosphat-Trihydrat | 0,04 g |
| Natrosol 250 HX (Hydroxyethylcellulose) | 1,75 g |
| Polysorbat 20 | 0,1 g |
| Paraffinöl dünnflüssig | 1,0 g |
| Wasser deionisiert ad 100 g | 96,76 g |

Die Herstellung des Hydrogels erfolgte nach dem bevorzugten Zweistufenverfahren:

### a) Herstellung der Voremulsion

In 80°C heißem Wasser wurden unter Rühren die Phosphate und das Konservierungsmittel Methylparaben gelöst und die Lösung anschließend auf Raumtemperatur abgekühlt. Im Paraffinöl wurde mittels eines schnelldrehenden Homogenisators der Emulgator Polysorbat 20 verteilt. Man gab nur langsam unter Rühren soviel Wasser zu, bis eine ca. 30%-ige grobe W/O Emulsion erhalten wurde. Diese Emulsion wurde kurz stehengelassen, wobei sie sich trennte. Nach erneutem Einschalten des Rührwerks wurde die Emulsion zur Phasenumkehr gebracht, wodurch eine sehr fein verteilte O/W-Emulsion erhalten wurde.

### b) Herstellung des Hydrogels

In die sterilfiltrierte Pufferlösung wurde die Paraffinölemulsion eingerührt und fein verteilt. Anschließend wurde mikrobiologisch reine Hydroxyethylcellulose in die Emulsion eingestreut und unter Rühren verteilt. Um eine vollständige Quellung zu erreichen, wurde das Gel 10-15 Stunden unter dem Laminar-Flow quellengelassen. Als letztes wurde die auf 4 mg/ml eingestellte IFN gamma Lösung langsam eingerührt. Die Abfüllung dieses Gemisches erfolgte unter Laminar-Air-flow-Bedingungen in sterile Tuben.

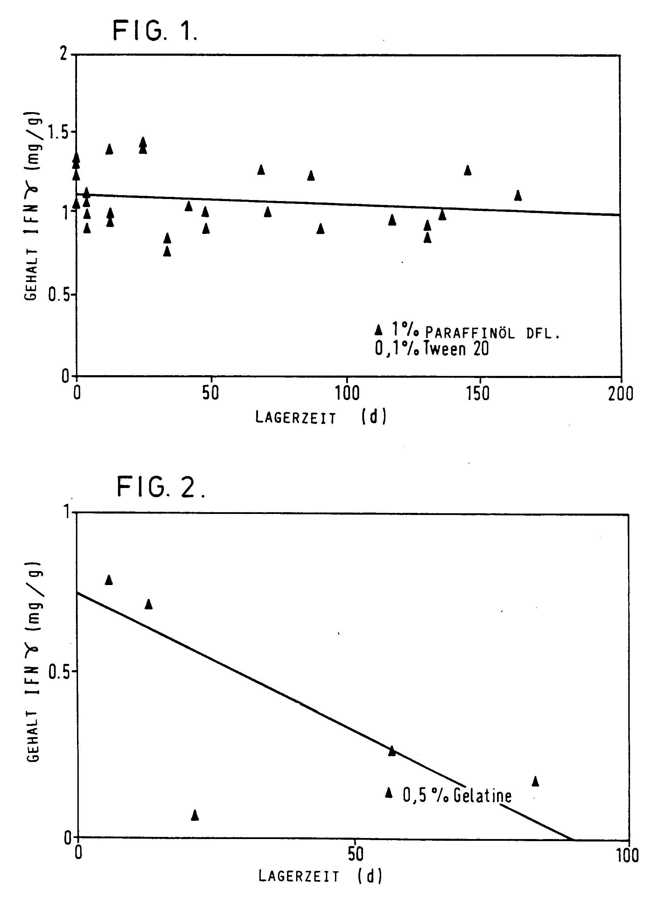

Der Verlauf der Lagerversuche ist in Fig. 1 dargestellt. Wie aus dem Diagramm ersichtlich, gewährleistet der Zusatz von Paraffinöl die Erhaltung der Aktivität des IFN-gamma, die mit Hilfe des ELISA-Tests gemessen wurde (die im ELISA-Test benutzten Antikörper binden biologisch aktives Protein, für die sie spezifisch sind); der im Diagramm dargestellte geringfügige Abfall ist angesichts der Teststreuung nicht signifikant.

Fig. 2 zeigt einen Vergleichsversuch mit Gelatine als Bestandteil einer Hydrogelformulierung ohne gesonderten Zusatz eines Stabilisators, aus dem die deutlich destabilisierende Wirkung von Gelatine auf IFN-gamma hervorgeht. Bei Verwendung von Gelatine als Hydrogelbildner ist demnach ein Zusatz von wirksamem Stabilisator unbedingt erforderlich.

Fig. 3 zeigt den Stabilitätsverlauf über einen Zeitraum von 15 Monaten (in dieser Darstellung-wie auch in den Fig. 4, 5, 6, - ist auf der Ordinate der Log. nat. der Konzentration des therapeutisch wirksamen Proteins aufgetragen).

### Beispiel 2

100 g Gel enthielten:

| | |
|---|---|
| IFN gamma | 0,1 g |
| Methylparaben | 0,2 g |
| Natriumdihydrogenphosphat-Monohydrat | 0,05 g |
| di-Kaliumhydrogenphosphat-Trihydrat | 0,04 g |
| Natrosol 250 HX | 1,75 g |
| Pluronic F68 | 0,1 g |
| Paraffinöl dünnflüssig | 1,0 g |
| Wasser deionisiert ad 100 g | 96,76 g |

In 80°C heißem Wasser wurden unter Rühren die Phosphate, das Konservierungsmittel Methylparaben und der Emulgator Pluronic F68 gelöst und die Lösung anschließend auf Raumtemperatur abgekühlt und sterilfiltriert. Mittels eines Homogenisators wurde das Paraffinöl eingebracht und verteilt. Danach erfolgte die Zugabe der Hydroxyethylcellulose unter Rühren im Vakuum. Als letztes erfolgte die Zugabe der auf 4 mg/ml eingestellten IFN gamma Lösung. Die Abfüllung erfolgte wie im Beispiel 1 angegeben.

### Beispiel 3

100 g Gel enthielten:

| | |
|---|---|
| TFN alpha | 0,1 g |
| Methylparaben | 0,213 g |
| Natriumdihydrogenphosphat-Monohydrat | 0,053 g |
| di-Kaliumhydrogenphosphat-Trihydrat | 0,0427 g |
| Natrosol 250 HX | 1,87 g |
| Polysorbat 20 | 0,107 g |
| Paraffinöl dünnflüssig | 1,07 g |
| Wasser deionisiert ad 100 g | 96,5443 g |

Die Herstellung des Hydrogels erfolgte wie im Beispiel 1 angegeben.

### Beispiel 4

100 g Gel enthielten:

| | |
|---|---|
| IFN alpha | 0,0005 g |
| Methylparaben | 0,2 g |
| Natriumdihydrogenphosphat-Monohydrat | 0,05 g |
| di-Kaliumhydrogenphosphat-Trihydrat | 0,04 g |
| Natrosol 250 HX | 1,75 g |
| Polysorbat 20 | 0,1 g |
| Paraffinöl dünnflüssig | 1,0 g |
| Wasser deionisiert ad 100 g | 96,8595 g |

Die Herstellung des Hydrogels erfolgte wie im Beispiel 1 angegeben.

### Beispiel 5

100 g Gel enthielten:

| | |
|---|---|
| IFN gamma | 0,100 g |
| Methylparaben | 0,2 g |
| Natriumdihydrogenphosphat-Monohydrat | 0,05 g |
| di-Kaliumhydrogenphosphat-Trihydrat | 0,04 g |
| Tauroglykolsäure | 0,01 g |
| Natrosol 250 HX | 1,75 g |
| Polysorbat 20 | 0,1 g |
| Paraffinöl dünnflüssig | 1,0 g |
| Wasser deionisiert ad 100 g | 96,75 g |

Die Herstellung des Hydrogels erfolgte wie im Beispiel 1, wobei der Permeationsbeschleuniger Tauroglykolsäure in die Pufferlösung eingerührt wurde.

### Beispiel 6

100 g Gel enthielten:

| | |
|---|---|
| IFN gamma | 0,05 g |
| Methylparaben | 0,2 g |
| Natriumdihydrogenphosphat-Monohydrat | 0,05 g |
| di-Kaliumhydrogenphosphat-Trihydrat | 0,04 g |
| Natrosol 250 HX | 1,75 g |
| Polysorbat 20 | 0,1 g |
| Paraffinöl dünnflüssig | 0,6 g |
| Paraffinöl dickflüssig | 0,4 g |
| Wasser deionisiert ad 100 g | 96,81 g |

Die Herstellung des Hydrogels erfolgte wie im Beispiel 1 angegeben.

### Beispiel 7

100 g Gel enthielten:

| | |
|---|---|
| IFN gamma | 0,05 g |
| Methylparaben | 0,2 g |
| Natriumdihydrogenphosphat-Monohydrat | 0,05 g |
| di-Kaliumhydrogenphosphat-Trihydrat | 0,04 g |
| Natrosol 250 HX | 1,75 g |
| Myristylalkohol | 1,0 g |
| Wasser deionisiert ad 100 g | 96,91 g |

Die Herstellung des Hydrogels erfolgte wie im Beispiel 2. Der Myristylalkohol wurde in der auf ca. 60°C erhitzten sterilfiltrierten Pufferlösung verteilt. Nach dem Abkühlen der Pufferlösung wurde wie im Beispiel 2 angegeben fortgefahren.

### Beispiel 8

100 Gramm Gelsubstanz enthalten:

| | |
|---|---|
| IFN gamma | 0.005 g |
| Methylparaben | 0.20 g |
| Succinatpuffer pH 6.00 | 0.0191 M |
| Natriumchlorid | 0.1435 M |
| Natrosol 250 HX | 1.75 g |
| Polysorbat 20 | 0.0952 g |
| Paraffinöl dünnflüssig | 0.952 |
| Wasser deionisiert | ad 100 g |

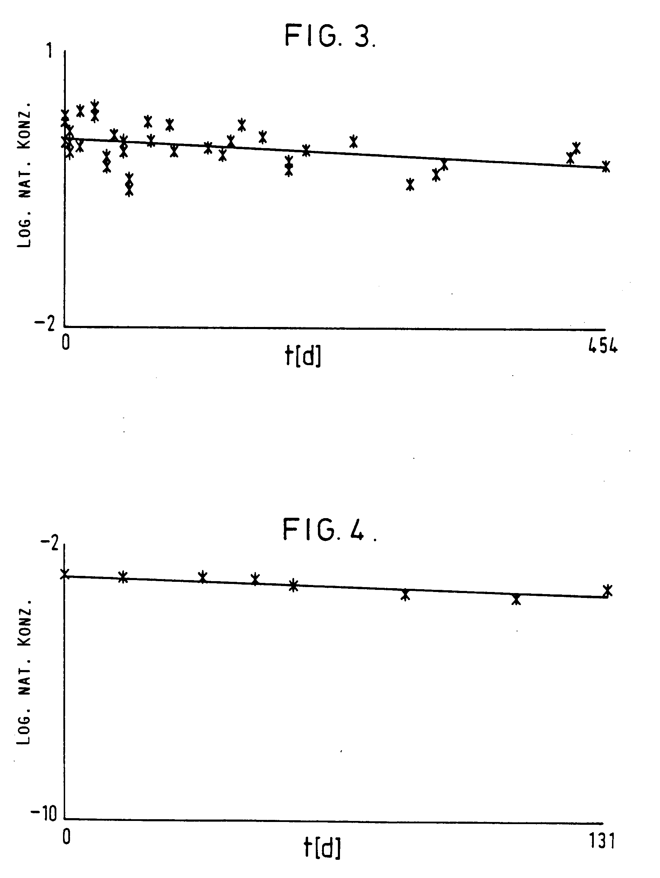

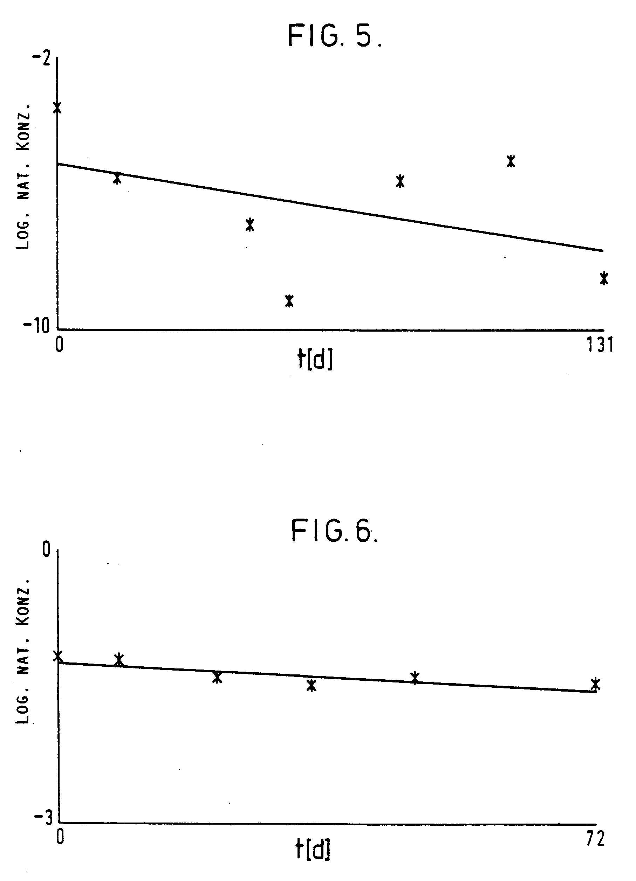

Die Herstellung des Hydrogeles erfolgte wie im Beispiel 1 angegeben. Der Stabilitätsverlauf ist in Fig. 4 dargestellt. Fig.5 zeigt den Verlauf eines Vergleichsversuches, bei dem dieselbe Formulierung ohne Zusatz von Paraffinöl verwendet wurde. Das Ergebnis des Vergleichsversuches zeigt einen Stabilitätsabfall bereits kurz nach der Herstellung.

### Beispiel 9

100 Gramm Gelsubstanz enthalten:

| | |
|---|---|
| IFN gamma | 0.025 g |
| Methylparaben | 0.20 g |
| Succinat (Puffer pH 6.2) | 0.2362 g |
| Natriumchlorid | 0.8766 g |
| Natrosol 250 HX | 1.75 g |
| Polysorbat 20 | 0.1 g |
| LABRAFIL 1944 CS | 1.0 g |
| Wasser deionisiert | ad 100 g |

Die Herstellung des Hydrogeles erfolgte wie im Beispiel 1 angeben

### Beispiel 10

100 Gramm Gelsubstanz enthalten:

| | |
|---|---|
| IFN gamma | 0.025 g |
| Methylparaben | 0.20 g |
| Succinat (Puffer pH 6.2) | 0.2362 g |
| Natriumchlorid | 0.8766 g |
| Natrosol 250 HX | 1.75 g |
| Polysorbat 20 | 0.1 g |
| LABRAFIL 2735 CS | 1.0 g |
| Wasser deionisiert | ad 100 g |

Die Herstellung des Hydrogeles erfolgte wie im Beispiel 1 angegeben.

### Beispiel 11

100 Gramm Gelsubstanz enthalten:

| | |
|---|---|
| IFN gamma | 0.025 g |
| Methylparaben | 0.20 g |
| Succinat (Puffer pH 6.2) | 0.2362 g |
| Natriumchlorid | 0.90 g |
| Natrosol 250 HX | 1.75 g |
| Polysorbat 20 | 0.1 g |
| Myristylalkohol | 1.0 g |
| Wasser deionisiert | ad 100 g |

Die Herstellung des Hydrogeles erfolgte folgendermaßen: Der Myristylalkohol wurde bei 50 - 60°C geschmolzen und anschließend die Voremulsion, wie in Beispiel 1 angegeben, jedoch bei 50 - 60°C, hergestellt. Die weitere Vorgangsweise entsprach Beispiel 1. Der Stabilitätsverlauf ist in Fig. 6 dargestellt.

### Beispiel 12

100 Gramm Gelsubstanz enthalten:

| | |
|---|---|
| TNF beta | 0.05 g |
| Methylparaben | 0.2 g |
| Natriumdihydrogenphosphat-Monohydrat | 0.05 g |
| di-Kaliumhydrogenphosphat-Trihydrat | 0.04 g |
| Natrosol 250 HX | 1.75 g |
| Polysorbat 20 | 0.2 g |
| Paraffinöl dünnflüssig | 2.0 g |
| Wasser deionisiert | ad 100 g |

Die Herstellung des Hydrogeles erfolgte wie im Beispiel 1 angegeben.

### Beispiel 13

100 Gramm Gelsubstanz enthalten:

| | |
|---|---|
| Lysozym | 2.4 Mio. Units |
| Methylparaben | 0.2 g |
| Natriumdihydrogenphosphat-Monohydrat | 0.05 g |
| di-Kaliumhydrogenphosphat-Trihydrat | 0.04 g |
| Natrosol 250 HX | 1.75 g |
| Polysorbat 20 | 0.2 g |
| Paraffinöl dünnflüssig | 2.0 g |
| Wasser deionisiert | ad 100 g |

Die Herstellung des Hydrogeles erfolgte wie im Beispiel 1 angegeben.

### Beispiel 14

100 Gramm Gelsubstanz enthalten:

| | |
|---|---|
| VAC alpha | 0.03 g |
| Methylparaben | 0.2 g |
| Natriumdihydrogenphosphat-Monohydrat | 0.05 g |
| di-Kaliumhydrogenphosphat-Trihydrat | 0.04 g |
| Natrosol 250 HX | 1.75 g |
| Polysorbat 20 | 0.1 g |
| Paraffinöl dünnflüssig | 1.0 g |
| Wasser deionisiert | ad 100 g |

Die Herstellung des Hydrogeles erfolgte wie im Beispiel 1 angegeben.

## Patentansprüche (Patentansprüche für folgende(n) Vertragsstaat(en): AT, BE, CH, LI, DE, FR, GB, IT, LU, NL, SE)

1. Pharmazeutische Zubereitung für die topische Anwendung, enthaltend ein oder mehrere stabilisierte therapeutisch wirksame Proteine sowie übliche Hilfs-, Träger- und Zusatzstoffe, dadurch gekennzeichnet, daß sie als Stabilisator fein verteilt eine oder mehrere physiologisch verträgliche hydrophobe Substanzen, insbesondere Paraffinöl(e), in einer das Protein stabilisierenden Menge enthält, die geringer ist als die Menge, in der die Substanz eine Trägerfunktion hat.

2. Pharmazeutische Zubereitung nach Anspruch 1, dadurch gekennzeichnet, daß sie ein natürliches oder rekombinantes menschliches oder tierisches Protein, ausgewählt aus der Gruppe Interferone, TNF-α, TNF-β, t-PA, einschließlich deren strukturell ähnlichen bioaktiven Äquivalenten einzeln oder in therapeutisch sinnvoller Mischung in therapeutisch wirksamer Menge enthält.

3. Pharmazeutische Zubereitung nach einem der vorhergehenden Ansprüche, dadurch gekennzeichnet, daß sie als hydrophobe Substanz Paraffinöl oder Mischungen von Paraffinölen in einer Menge von 0.1 bis 3 %, bezogen auf die Gesamtmasse, enthält.

4. Pharmazeutische Zubereitung nach einem der vorhergehenden Ansprüche, dadurch gekennzeichnet, daß sie zusätzlich zur fein verteilten hydrophoben Substanz einen, insbesondere nichtionischen, Emulgator enthält.

5. Pharmazeutische Zubereitung nach einem der vorhergehenden Ansprüche, dadurch gekennzeichnet, daß sie als Hydrogel vorliegt.

6. Pharmazeutische Zubereitung nach Anspruch 5, dadurch gekennzeichnet, daß sie einen Hydrogelbildner aus der Gruppe Gelatine, Cellulosederivate wie Hydroxyethylcellulose, oder synthetische Polymere wie Polyvinylalkohol, einzeln oder in Mischung enthält.

7. Pharmazeutische Zubereitung nach Anspruch 6, dadurch gekennzeichnet, daß sie als Hydrogelbildner Hydroxyethylcellulose enthält.

8. Pharmazeutische Zubereitung nach einem der vorhergehenden Ansprüche, dadurch gekennzeichnet, daß sie ein physiologisch verträgliches Konservierungsmittel, wie p-Hydroxybenzoesäureester, Sorbinsäure, Chlorhexidindigluconat, Benzalkoniumchlorid und Hexadecyltrimethylammoniumbromid einzeln oder in Mischung enthält.

9. Pharmazeutische Zubereitung nach einem der vorhergehenden Ansprüche, dadurch gekennzeichnet, daß sie als weitere Zusatzstoffe Permeationsbeschleuniger und/oder Feuchthaltemittel und/oder ein Puffersystem enthält.

10. Verfahren zur Herstellung einer pharmazeutischen Zubereitung nach einem der Ansprüche 1 bis 9, dadurch gekennzeichnet, daß man in einer ersten Verfahrensstufe im System Stabilisator/Wasser/gegebenenfalls Emulgator eine Phasenumkehr von einer W/O-Emulsion zu einer O/W-Emulsion herbeiführt und die dabei erhaltene feine Voremulsion mit der Hauptmenge der wässerigen Phase vereinigt.

11. Verfahren nach Anspruch 10, dadurch gekennzeichnet, daß man zunächst eine Voremulsion herstellt, indem man zum Stabilisator und dem darin gegebenenfalls enthaltenen fein verteilten Emulgator Wasser bis zum Erhalt einer groben W/O-Emulsion unter Rühren zugibt, den Rührvorgang bis zur Sedimentation der Emulsion unterbricht, anschließend durch neuerliches Rühren und weitere Wasserzugabe bis zu einem Anteil von ca. 50 % unter Phasenumkehr eine feine O/W-Emulsion herstellt, diese Voremulsion in der gegebenenfalls Konservierungsmittel und weitere Zusatzstoffe enthaltenden Pufferlösung fein verteilt, den Hydrogelbildner einbringt und quellen läßt und abschließend die Proteinlösung zufügt.

12. Verwendung von physiologisch verträglichen hydrophoben Substanzen zur Stabilisierung von therapeutisch wirksamen Proteinen in pharmazeutischen Zubereitungen für die topische Anwendung, mit der Maßgabe, daß die hydrophobe Substanz in der Zubereitung in einer Menge enthalten ist, die geringer ist als die Menge, in der die Substanz eine Trägerfunktion hat.

13. Verwendung nach Anspruch 12 mit der Maßgabe, daß die hydrophobe Substanz Paraffinöl ist.

14. Verwendung nach einem der Ansprüche 12 und 13 mit der Maßgabe, daß die pharmazeutische Zubereitung als Hydrogel vorliegt.

## Patentansprüche (Patentansprüche für folgende(n) Vertragsstaat(en): ES, GR)

1. Verfahren zur Herstellung einer topisch anzuwendenden pharmazeutischen Zubereitung in Form einer O/W-Emulsion, dadurch gekennzeichnet, daß man in einer wäßrigen Phase, enthaltend ein oder mehrere therapeutisch wirksame Proteine sowie übliche Hilfs-, Träger- und Zusatzstoffe, eine oder mehrere physiologisch verträgliche hydrophobe Substanzen, insbesondere Paraffinöl(e), in einer das Protein stabilisierenden Menge, die geringer ist als die Menge, in der die Substanz eine Trägerfunktion hat, fein verteilt.

2. Verfahren nach Anspruch 1, dadurch gekennzeichnet, daß die pharmazeutische Zubereitung ein natürliches oder rekombinantes menschliches oder tierisches Protein, ausgewählt aus der Gruppe Interferone, TNF-α, TNF-β, t-PA, einschließlich deren strukturell ähnlichen bioaktiven Äquivalenten einzeln oder in therapeutisch sinnvoller Mischung in therapeutisch wirksamer Menge enthält.

3. Verfahren nach Anspruch 1 oder 2, dadurch gekennzeichnet, daß die pharmazeutische Zubereitung als hydrophobe Substanz Paraffinöl oder Mischungen von Paraffinölen in einer Menge von 0.1 bis 3 %, bezogen auf die Gesamtmasse, enthält.

4. Verfahren nach einem der vorhergehenden Ansprüche, dadurch gekennzeichnet, daß die pharmazeutische Zubereitung zusätzlich zur fein verteilten hydrophoben Substanz einen, insbesondere nichtionischen, Emulgator enthält.

5. Verfahren nach einem der vorhergehenden Ansprüche, dadurch gekennzeichnet, daß die pharmazeutische Zubereitung als Hydrogel vorliegt.

6. Verfahren nach Anspruch 5, dadurch gekennzeichnet, daß die pharmazeutische Zubereitung einen Hydrogelbildner aus der Gruppe Gelatine, Cellulosederivate wie Hydroxyethylcellulose, oder synthetische Polymere wie Polyvinylalkohol, einzeln oder in Mischung enthält.

7. Verfahren nach Anspruch 6, dadurch gekennzeichnet, daß die pharmazeutische Zubereitung als Hydrogelbildner Hydroxyethylcellulose enthält.

8. Verfahren nach einem der vorhergehenden Ansprüche, dadurch gekennzeichnet, daß die pharmazeutische Zubereitung ein physiologisch verträgliches Konservierungsmittel, wie p-Hydroxybenzoesäureester, Sorbinsäure, Chlorhexidindigluconat, Benzalkoniumchlorid und Hexadecyltrimethylammoniumbromid einzeln oder in Mischung enthält.

9. Verfahren nach einem der vorhergehenden Ansprüche, dadurch gekennzeichnet, daß die pharmazeutische Zubereitung als weitere Zusatzstoffe Permeationsbeschleuniger und/oder Feuchthaltemittel und/oder ein Puffersystem enthält.

10. Verfahren nach einem der Ansprüche 1 bis 9, dadurch gekennzeichnet, daß man in einer ersten Verfahrensstufe im System hydrophobe Substanz(en)/Wasser/gegebenenfalls Emulgator eine Phasenumkehr von einer W/O-Emulsion zu einer O/W-Emulsion herbeiführt und die dabei erhaltene feine Voremulsion mit der Hauptmenge der wäßrigen Phase vereinigt.

11. Verfahren nach Anspruch 10, dadurch gekennzeichnet, daß man zunächst eine Voremulsion herstellt, indem man zur hydrophoben Substanz und dem darin gegebenenfalls enthaltenen fein verteilten Emulgator Wasser bis zum Erhalt einer groben W/O-Emulsion unter Rühren zugibt, den Rührvorgang bis zur Sedimentation der Emulsion unterbricht, anschließend durch neuerliches Rühren und weitere Wasserzugabe bis zu einem Anteil von ca. 50 % unter Phasenumkehr eine feine O/W-Emulsion herstellt, diese Voremulsion in der gegebenenfalls Konservierungsmittel und weitere Zusatzstoffe enthaltenden Pufferlösung fein verteilt, den Hydrogelbildner einbringt und quellen läßt und abschließend die Proteinlösung zufügt.

12. Verwendung von physiologisch verträglichen hydrophoben Substanzen zur Stabilisierung von therapeutisch wirksamen Proteinen in pharmazeutischen Zubereitungen für die topische Anwendung, mit der Maßgabe, daß die hydrophobe Substanz in der Zubereitung in einer Menge enthalten ist, die geringer ist als die Menge, in der die Substanz eine Trägerfunktion hat.

13. Verwendung nach Anspruch 12 mit der Maßgabe, daß die hydrophobe Substanz Paraffinöl ist.

14. Verwendung nach einem der Ansprüche 12 und 13 mit der Maßgabe, daß die pharmazeutische Zubereitung als Hydrogel vorliegt.

## Claims (Claims for the following Contracting State(s): AT, BE, CH, LI, DE, FR, GB, IT, LU, NL, SE)

1. A pharmaceutical preparation for topical application containing one or more stabilized, therapeutically active proteins and conventional excipients, carriers and additives, characterised in that it contains as stabiliser one or more physiologically acceptable hydrophobic substances, particularly paraffin oil or oils, in finely divided form, in an amount which stabilises the protein and is less than the amount in which the substance has a carrier function.

2. A pharmaceutical preparation according to claim 1, characterised in that it contains a natural or recombinant, human or animal protein, selected from the group comprising the interferons, TNFα, TNFβ, t-PA, including the structurally similar bioactive equivalents thereof, individually or in a therapeutically useful mixture, in a therapeutically active quantity.

3. A pharmaceutical preparation according to one of the preceding claims, characterised in that it contains as hydrophobic substance paraffin oil or mixtures of paraffin oils in a quantity of from 0.1 to 3%, based on the total mass.

4. A pharmaceutical preparation according to one of the preceding claims, characterised in that, in addition to the finely distributed hydrophobic substance, it contains an emulsifier, more particularly a non-ionic emulsifier.

5. A pharmaceutical preparation according to one of the preceding claims, characterised in that it is in the form of a hydrogel.

6. A pharmaceutical preparation according to claim 5, characterised in that it contains a hydrogel forming agent from the group of gelatines, cellulose derivatives such as hydroxyethylcellulose, or synthetic polymers such as polyvinyl alcohol, either individually or in admixture.

7. A pharmaceutical preparation according to claim 6, characterised in that it contains hydroxyethyl-cellulose as hydrogel forming agent.

8. A pharmaceutical preparation according to one of the preceding claims, characterised in that it contains a physiologically acceptable preservative such as p-hydroxybenzoate, sorbic acid, chlorhexidine digluconate, benzalkonium chloride and hexadecyltrimethyl ammonium bromide, either individually or in admixture.

9. A pharmaceutical preparation according to one of the preceding claims, characterised in that it contains, as further additives, permeation accelerators and/or moisture retaining agents and/or a buffer system.

10. A process for preparing a pharmaceutical preparation according to one of claims 1 - 9, characterised in that, in a first step of the process, in the system comprising the stabiliser/water/optionally emulsifier, phase inversion is effected from a W/O emulsion to an O/W emulsion and the fine pre-emulsion thus obtained is combined with the majority of the aqueous phase.

11. A process according to claim 10, characterised in that, first of all, a pre-emulsion is prepared by adding water to the stabiliser and the finely divided emulsifier optionally contained therein, with stirring, until a coarse W/O emulsion is obtained, the stirring process is stopped until the emulsion has sedimented, then by the resumption of stirring and the addition of more water to give a content of about 50%, with phase inversion, a fine O/W emulsion is produced, this pre-emulsion is finely divided in the buffer solution, which optionally contains preservatives and other additives, the hydrogel forming agent is introduced and allowed to swell and finally the protein solution is added.

12. Use of physiologically acceptable hydrophobic substances for stabilising therapeutically active proteins in pharmaceutical preparations for topical application, with the proviso that the hydrophobic substance is contained in the preparation in an amount which is less than the amount in which the substance has a carrier function.

13. Use according to claim 12, with the proviso that the hydrophobic substance is paraffin oil.

14. Use according to one of claims 12 and 13, with the proviso that the pharmaceutical preparation is in the form of a hydrogel.

## Claims (Claims for the following Contracting State(s): ES, GR)

1. A process for preparing a pharmaceutical preparation for topical application in the form of an O/W emulsion, characterised in that one or more physiologically acceptable hydrophobic substances, more particularly paraffin oil or oils, in an amount which stabilises the protein and is less than the amount in which the substance has a carrier function, are finely distributed in an aqueous phase containing one or more therapeutically active proteins as well as conventional excipients, carriers and additives.

2. A process according to claim 1, characterised in that the pharmaceutical preparation contains a natural or recombinant, human or animal protein, selected from the group comprising the interference, TNFα, TNFβ, t-PA, including the structurally similar bioactive equivalents thereof, individually or in a therapeutically useful mixture, in a therapeutically active quantity.

3. A process according to claim 1 or 2, characterised in that the pharmaceutical preparation contains as hydrophobic substance paraffin oil or mixtures of paraffin oils in a quantity of from 0.1 to 3%, based on the total mass.

4. A process according to one of the preceding claims, characterised in that, in addition to the finely distributed hydrophobic substance, the pharmaceutical preparation contains an emulsifier, more particularly a non-ionic emulsifier.

5. A process according to one of the preceding claims, characterised in that the pharmaceutical preparation is in the form of a hydrogel.

6. A process according to claim 5, characterised in that the pharmaceutical preparation contains a hydrogel forming agent selected from the group of gelatines, cellulose derivatives such as hydroxyethylcellulose, or synthetic polymers such as polyvinyl alcohol, either individually or in admixture.

7. A process according to claim 6, characterised in that the pharmaceutical preparation contains hydroxyethylcellulose as hydrogel forming agent.

8. A process according to one of the preceding claims, characterised in that the pharmaceutical preparation contains a physiologically acceptable preservative such as p-hydroxybenzoate, sorbic acid, chlorhexidine digluconate, benzalkonium chloride and hexadecyltrimethyl ammonium bromide either individually or in admixture.

9. A process according to one of the preceding claims, characterised in that the pharmaceutical preparation contains, as further additives, permeation accelerators and/or moisture retaining agents and/or a buffer system.

10. A process according to one of claims 1 to 9, characterised in that, in a first step of the process, in the system comprising hydrophobic substance(s)/water/optionally emulsifier, phase inversion is effected from a W/O emulsion to an O/W emulsion and the fine pre-emulsion obtained is combined with the majority of the aqueous phase.

11. A process according to claim 10, characterised in that, first of all, a pre-emulsion is prepared by adding water to the hydrophobic substance and the finely divided emulsifier, optionally contained therein, with stirring, the stirring process is stopped until the emulsion has sedimented, then by the resumption of stirring and the addition of more water to give a content of about 50%, with phase inversion, a fine O/W emulsion is produced, this pre-emulsion is finely divided in the buffer solution, which optionally contains preservatives and other additives, the hydrogel forming agent is introduced and allowed to swell and finally the protein solution is added.

12. Use of physiologically acceptable hydrophobic substances for stabilising therapeutically active proteins in pharmaceutical preparations for topical application, with the proviso that the hydrophobic substance is contained in the preparation in an amount which is less than the amount in which the substance has a carrier function.

13. Use according to claim 12, with the proviso that the hydrophobic substance is paraffin oil.

14. Use according to one of claims 12 and 13, with the proviso that the pharmaceutical preparation is in the form of a hydrogel.

## Revendications (Revendications pour l'(les) Etat(s) contractant(s) suivant(s): AT, BE, CH, LI, DE, FR, GB, IT, LU, NL, SE)

1. Préparation pharmaceutique à usage topique, contenant une ou plusieurs protéines thérapeutiquement efficaces stabilisées et des adjuvants, supports et additifs habituels, caractérisée en ce qu'elle contient comme stabilisant, de manière finement répartie, une ou plusieurs substances hydrophobes physiologiquement acceptables, en particulier une ou des huiles de paraffine, en une quantité stabilisant la protéine qui est inférieure à la quantité pour laquelle la substance a une fonction de support.

2. Préparation pharmaceutique selon la revendication 1, caractérisée en ce qu'elle contient une protéine humaine ou animale naturelle ou recombinante choisie dans le groupe des interférons, de TNF-α, de TNF-β, de t-PA, y compris leurs équivalents bioactifs analogues du point de vue structural, seule ou en mélange thérapeutiquement judicieux en une quantité thérapeutiquement efficace.

3. Préparation pharmaceutique selon l'une des revendications précédentes, caractérisée en ce qu'elle contient comme substance hydrophobe une huile de paraffine ou des mélanges d'huiles de paraffine en une quantité de 0,1 à 3% par rapport à la masse totale.

4. Préparation pharmaceutique selon l'une des revendications précédentes, caractérisée en ce qu'elle contient en plus de la substance hydrophobe finement répartie un émulsifiant, en particulier non ionique.

5. Préparation pharmaceutique selon l'une des revendications précédentes, caractérisée en ce qu'elle est sous forme d'hydrogel.

6. Préparation pharmaceutique selon la revendication 5, caractérisée en ce qu'elle contient un formateur d'hydrogel du groupe de la gélatine, des dérivés cellulosiques comme l'hydroxyéthylcellulose, ou des polymères synthétiques comme le poly(alcool vinylique), seul ou en mélange.

7. Préparation pharmaceutique selon la revendication 6, caractérisée en ce qu'elle contient comme formateur d'hydrogel de l'hydroxyéthylcellulose.

8. Préparation pharmaceutique selon l'une des revendications précédentes, caractérisée en ce qu'elle contient un conservateur physiologiquement acceptable comme un ester d'acide p-hydroxybenzoïque, l'acide sorbique, le digluconate de chlorhexidine, le chlorure de benzalkonium et le bromure d'hexadécyltriméthylammonium seul ou en mélange.

9. Préparation pharmaceutique selon l'une des revendications précédentes, caractérisée en ce qu'elle contient comme additifs supplémentaires des accélérateurs de perméation et/ou des agents maintenant l'humidité et/ou un système tampon.

10. Procédé d'obtention d'une préparation pharmaceutique selon l'une des revendications 1 à 9, caractérisé en ce que, dans une première étape de procédé, on provoque dans le système stabilisant/eau/éventuellement émulsifiant une inversion de phases d'une émulsion E/H à une émulsion H/E et on combine la fine pré-émulsion ainsi obtenue à la quantité principale de la phase aqueuse.

11. Procédé selon la revendication 10, caractérisé en ce que l'on prépare tout d'abord une pré-émulsion en ajoutant sous agitation au stabilisant et à l'émulsifiant finement réparti éventuellement contenu dans celui-ci de l'eau jusqu'à ce que l'on obtienne une émulsion E/H grossière, on interrompt le processus d'agitation jusqu'à la sédimentation de l'émulsion, puis on prépare une fine émulsion H/E par une nouvelle agitation et une addition d'eau supplémentaire jusqu'à une proportion d'environ 50% avec inversion de phases, on répartit finement cette pré-émulsion dans la solution tampon contenant éventuellement des conservateurs et d'autres additifs, on introduit et laisse gonfler le formateur d'hydrogel et enfin on ajoute la solution de protéine.

12. Utilisation de substances hydrophobes physiologiquement acceptables pour la stabilisation de protéines thérapeutiquement efficaces dans des préparations pharmaceutiques à usage topique, dans laquelle la substance hydrophobe est contenue dans la préparation en une quantité qui est inférieure à la quantité pour laquelle la substance a une fonction de support.

13. Utilisation selon la revendication 12, dans laquelle la substance hydrophobe est une huile de paraffine.

14. Utilisation selon l'une des revendications 12 et 13, dans laquelle la préparation pharmaceutique est sous forme d'hydrogel.

## Revendications (Revendications pour l'(les) Etat(s) contractant(s) suivant(s): ES, GR)

1. Procédé d'obtention d'une préparation pharmaceutique à usage topique sous forme d'une émulsion H/E, caractérisé en ce que, dans une phase aqueuse contenant une ou plusieurs protéines thérapeutiquement efficaces et des adjuvants, supports et additifs habituels, on répartit finement une ou plusieurs substances hydrophobes physiologiquement acceptables, en particulier une ou des huiles de paraffine, en une quantité stabilisant la protéine qui est inférieure à la quantité pour laquelle la substance a une fonction de support.

2. Procédé selon la revendication 1, caractérisé en ce que la préparation pharmaceutique contient une protéine humaine ou animale naturelle ou recombinante choisie dans le groupe des interférons, de TNF-α, de TNF-β, de t-PA, y compris leurs équivalents bioactifs analogues du point de vue structural, seule ou en mélange thérapeutiquement judicieux en une quantité thérapeutiquement efficace.

3. Procédé selon la revendication 1 ou 2, caractérisé en ce que la préparation pharmaceutique contient comme substance hydrophobe une huile de paraffine ou des mélanges d'huiles de paraffine en une quantité de 0,1 à 3% par rapport à la masse totale.

4. Procédé selon l'une des revendications précédentes, caractérisé en ce que la préparation pharmaceutique contient en plus de la substance hydrophobe finement répartie un émulsifiant, en particulier non ionique.

5. Procédé selon l'une des revendications précédentes, caractérisé en ce que la préparation pharmaceutique est sous forme d'hydrogel.

6. Procédé selon la revendication 5, caractérisé en ce que la préparation pharmaceutique contient un formateur d'hydrogel du groupe de la gélatine, des dérivés cellulosiques comme l'hydroxyéthylcellulose, ou des polymères synthétiques comme le poly(alcool vinylique), seul ou en mélange.

7. Procédé selon la revendication 6, caractérisé en ce que la préparation pharmaceutique contient comme formateur d'hydrogel de l'hydroxyéthylcellulose.

8. Procédé selon l'une des revendications précédentes, caractérisé en ce que la préparation pharmaceutique contient un conservateur physiologiquement acceptable comme un ester d'acide p-hydroxybenzoïque, l'acide sorbique, le digluconate de chlorhexidine, le chlorure de benzalkonium et le bromure d'hexadécyltriméthylammonium seul ou en mélange.

9. Procédé selon l'une des revendications précédentes, caractérisé en ce que la préparation pharmaceutique contient comme additifs supplémentaires des accélérateurs de perméation et/ou des agents maintenant l'humidité et/ou un système tampon.

10. Procédé selon l'une des revendications 1 à 9, caractérisé en ce que, dans une première étape de procédé, on provoque dans le système substance(s) hydrophobe(s)/eau/éventuellement émulsifiant une inversion de phases d'une émulsion E/H à une émulsion H/E et on combine la fine pré-émulsion ainsi obtenue à la quantité principale de la phase aqueuse.

11. Procédé selon la revendication 10, caractérisé en ce que l'on prépare tout d'abord une pré-émulsion en ajoutant sous agitation à la substance hydrophobe et à l'émulsifiant finement réparti éventuellement contenu dans celle-ci de l'eau jusqu'à ce que l'on obtienne une émulsion E/H grossière, on interrompt le processus d'agitation jusqu'à la sédimentation de l'émulsion, puis on prépare une fine émulsion H/E par une nouvelle agitation et une addition d'eau supplémentaire jusqu'à une proportion d'environ 50% avec inversion de phases, on répartit finement cette pré-émulsion dans la solution tampon contenant éventuellement des conservateurs et d'autres additifs, on introduit et laisse gonfler le formateur d'hydrogel et enfin on ajoute la solution de protéine.

12. Utilisation de substances hydrophobes physiologiquement acceptables pour la stabilisation de protéines thérapeutiquement efficaces dans des préparations pharmaceutiques à usage topique, dans laquelle la substance hydrophobe est contenue dans la préparation en une quantité qui est inférieure à la quantité pour laquelle la substance a une fonction de support.

13. Utilisation selon la revendication 12, dans laquelle la substance hydrophobe est une huile de paraffine.

14. Utilisation selon l'une des revendications 12 et 13, dans laquelle la préparation pharmaceutique est sous forme d'hydrogel.
